# EUROPÄISCHE PATENTSCHRIFT

(11) **EP 1 296 601 B1**
(45) Veröffentlichungstag und Bekanntmachung des Hinweises auf die Patenterteilung: **02.08.2006**
(21) Anmeldenummer: 01937926.2
(22) Anmeldetag: 19.06.2001
(51) Int. Cl.: A61B 17/32, B26F 3/00

(54) **EINRICHTUNG ZUR ERZEUGUNG EINES FLÜSSIGKEITSSTRAHLES FÜR DAS ENTFERNEN INSBESONDERE VON BIOLOGISCHEM GEWEBE**
DEVICE FOR PRODUCING A LIQUID JET FOR REMOVING ESPECIALLY BIOLOGICAL TISSUE
DISPOSITIF DE GENERATION D'UN JET LIQUIDE POUR ELIMINER NOTAMMENT DU TISSU BIOLOGIQUE

(30) Priorität: 20.06.2000 CH 121300
(43) Veröffentlichungstag der Anmeldung: 02.04.2003
(73) Patentinhaber: MEDAXIS AG, 5000 Aarau (CH)
(72) Erfinder: AMMANN, Roger, CH-5018 Erlinsbach (CH)
(74) Vertreter: Luchs, Willi
(86) Internationale Anmeldenummer: PCT/CH2001/000385
(87) Internationale Veröffentlichungsnummer: WO 2001/097700

(56) Entgegenhaltungen:
- EP-A- 0 232 678
- WO-A-99/33510
- DE-U- 9 200 447
- DE-U- 29 913 886
- US-A- 5 871 462
- US-A- 6 068 640

## Beschreibung

Die Erfindung bezieht sich auf eine Einrichtung zur Erzeugung eines Flüssigkeitsstrahles für das Entfernen insbesondere von biologischem Gewebe, mit einem Gerät, in welchem ein Pumpenaggregat zur Erzeugung eines Hochdruckes der Flüssigkeit in einem Bereich bis zu 800 bar angeordnet ist, an welches ein Druckversorgungsmittel und über eine flexible Verbindungsleitung eine den Flüssigkeitsstrahl erzeugende Düse anschliessbar ist, welche bei der Austrittsöffnung einen Lochdurchmesser zwischen 20 und 120 Mikrometern aufweist.

Bei einer gattungsmässigen chirurgischen Einrichtung nach der Druckschrift EP-A-0 232 678 ist ein Pumpenaggregat vorgesehen, welches einen Druck erzeugt und über eine biegsame metallische Leitung mit einer den Flüssigkeitsstrahl auslassenden Düse verbunden ist, wobei mit ihr ein Strahl mit einer Anfangsgeschwindigkeit gleich oder grösser als die Machzahl erzeugt wird. Dieses Pumpenaggregat sowie die übrigen zugehörigen Bestandteile sind üblicherweise in einem Gehäuse angeordnet und darin fixiert. Da eine solche Flüssigkeitsstrahl-Schneideinrichtung insbesondere für die Chirurgie verwendet wird, wie beispielsweise bei Leberoperationen zur Zerstörung eines Leberparenchyms oder ähnlichem, ist dieses Gerät zusammen mit der als Kompressor ausgebildeten Druckversorgung für das Pumpenaggregat entsprechend massiv und schwer ausgebildet,

In der Druckschrift DE-A-299 13 886 ist eine Gartenschere beschrieben, welche mindestens eine Hochdruckpumpe und eine Düse aufweist, aus welcher ein Wasserstrahl mit hoher Geschwindigkeit austreten soll, um Gartenpflanzen aus der Nähe zu schneiden. Es ist jedoch nicht vorgesehen, einen solchen Hochdruck zu erzeugen, mittels dem biologisches Gewebe getrennt werden könnte.

Die beiden Druckschriften WO-A-99/33510 und U5,871,462 offenbaren jeweils ein Gerät zum Erzeugen eines Hochdruck-Wasserstrahls. Es ist aber in keinem der Geräte vorgesehen, mit einem Druck von 800 bar zu arbeiten. Die verwendeten Kurbeitriebe zum Antrieb eines Hochdruckkolbens müssen beim Vorsehen eines hohen Druckes massiv gebaut sein. Diese Geräte eignen sich daher nicht als Mobilgeräte.

Der vorliegenden Erfindung wurde demgegenüber die Aufgabe zugrundegelegt, eine Einrichtung nach der eingangs erwähnten Gattung zu schaffen, die sich insbesondere auch für die ambulante Behandlung mit einer für das Bedienungspersonal von Hand transportierbaren Ausführung eignet. Darüberhinaus soll diese Einrichtung für neue Verwendungszwecke von Behandlungen eingesetzt werden können, welche nicht nur von Ärzten. Medizinern, sondern auch von Pflegern, paramedizinischem Personal oder ähnlichen durchgeführt werden können.

Die Aufgabe ist erfindungsgemäss dadurch gelöst, dass das Gerät und das separat zu diesem vorgesehene Druckversorgungsmittel für das Pumpenaggregat jeweils ein solches Gewicht aufweisen, dass sie sich für eine mobile Anwendung eignen und demgemäss jeweils von Hand tragbar sind wobei das Pumpenaggregat als Differentialkolbenpumpe ausgebildet ist.

Mit dieser erfindungsgemässen Einrichtung können Behandlungen auf ambulantem Wege vorgenommen werden, bei denen man praktisch unabhängig von den Ortlichkeiten ist, was mit den bisherigen Einrichtungen nicht machbar war.

Die Aufgabe ist des weiteren erfindungsgemäss dadurch gelöst, dass mit diesem Flüssigkeitsstrahl Wundreinigungen oder ein Wundmanagement bei Verbrennungen, Geschwüren, Dekubitus oder ähnlichem vorgenommen werden können.

Durch die transportable Ausbildung der Einrichtung können somit Patienten auch ausserhalb von Operationssälen behandelt werden, gerade auch solche, die sich nicht in Spitälern aufhalten.

Es hat sich in überraschender Weise herausgestellt, dass Wunden mit der Behandlung durch einen Flüssigkeitsstrahl schneller heilen als auf herkömmlicher Basis.

Ein Ausführungsbeispiel der Erfindung sowie weitere Vorteile derselben sind nachfolgend anhand der Zeichnung näher erläutert. Es zeigt:

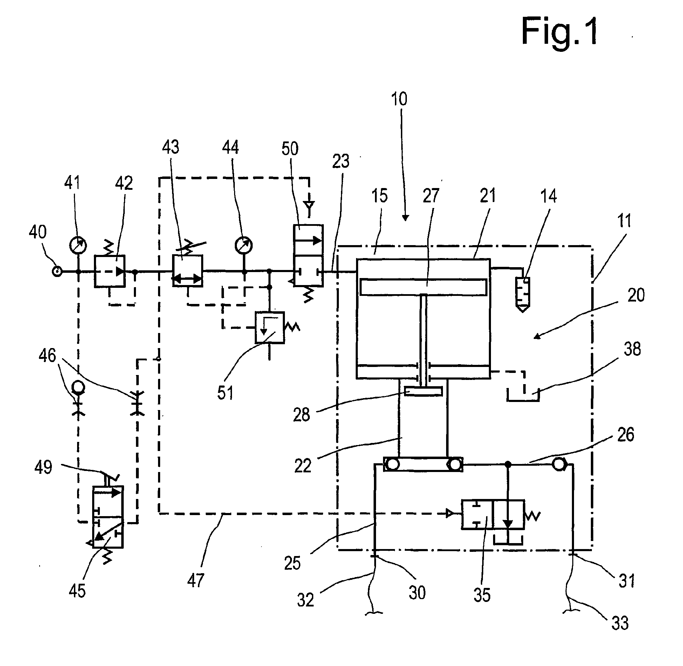

- Fig.1: ein Blockschema einer erfindungsgemässen Einrichtung.

Fig.1 zeigt ein Blockschema einer Einrichtung 10 zur Erzeugung eines Flüssigkeitsstrahles für das Entfernen insbesondere von biologischem Gewebe. Diese Einrichtung 10 eignet sich sehr vorteilhaft zum Herausschneiden von Gewebeteilen, insbesondere von parenchymatösem Gewebe, wie beispielsweise bei Leber-, Nieren- oder ähnlichen Operationen.

Die Einrichtung 10 weist ein Gehäuse 11 auf, in welchem ein Pumpenaggregat 20 zur Erzeugung eines Hochdruckes der Schneidflüssigkeit angeordnet ist. Dieses Pumpenaggregat 20 ist als eine an sich herkömmliche Differentialkolbenpumpe ausgebildet, die mit Druckluft von einem Druckluftnetz oder einer separaten Druckluft-Erzeugungseinrichtung gespeist wird. Die Pumpe 20 besteht aus einem ersten grösseren Zylinder 21 als Niederdruckteil, einem in diesem hin- und her bewegbar geführten Antriebskolben 27 und einem zweiten kleineren, koaxial zum ersteren angeordneten Hochdruckzylinder 22, in dem ein mit dem Antriebskolben 27 verbundener Plungerkolben 28 enthalten ist. In den grösseren Zylinder 21 führt die Druckluftzufuhr 23 bzw. ein Auslassventil 24, derweil beim Hochdruckzylinder 22 eine Zufuhr- 25 und eine Auslassleitung 26 für die Flüssigkeit vorhanden sind. Diese Zufuhr- 25 und Auslassleitung 26 führen jeweils zu einem von ausserhalb des Gehäuses 11 zugänglichen Anschlussstecker 30, 31, wobei der Stecker 30 ein mit einem Flüssigkeitsbehälter verbundenen Schlauch 32, indessen der Stecker 31 an eine flexible Metalleitung 33 anschliessbar ist. Im Flüssigkeitsbehälter ist eine physiologische Lösung enthalten, zum Beispiel eine sterile Kochsalzlösung mit ca. 0.9% Anteil Kochsalz oder dergleichen. Am Ende dieser flexiblen Metalleitung 33 ist die den Flüssigkeitsstrahl erzeugende Düse befestigt, welche mit einem Handgriff versehen ist und auf diese Art eine manuelle Betätigung mit diesem Schneidgerät erfolgt.

Erfindungsgemäss weisen das Gerät und das separat zu diesem vorgesehene Druckversorgungsmittel für das Pumpenaggregat 20 jeweils ein solches Gewicht auf, dass sie sich für eine mobile Anwendung eignen und demgemäss jeweils von Hand tragbar sind.

Des weiteren können gerade aufgrund der mobilen Ausbildung der Einrichtung mit diesem Flüssigkeitsstrahl in sehr wirtschaftlicher Weise Wundreinigungen oder ein Wundmanagement bei Verbrennungen, Geschwüren, Dekubitus oder ähnlichem vorgenommen werden.

Beim hochdruckseitigen Ausgang des Pumpenaggregates 20 ist ferner ein Druckentlastungsventil 35 vorgesehen, welches über eine Verbindungsleitung 36 an die Auslassleitung 26 der Pumpe angeschlossen ist.

Das gesamte Pumpenaggregat 20 ist vorteilhaft auf einem im strichpunktiert angedeuteten Gehäuse 11 angeordneten Tragelement 15 gehalten. Dazu gehören die Differentialkolbenpumpe mit den Zylindern 21, 22 und den darin angeordneten Kolben 27, 28, die hochdruckseitige Zufuhr- 25 und Auslassleitung 26 als Verbindungsleitungen mit den Anschlussstekkern 30, 31 und das pneumatisch betätigbare Druckentlastungsventil 35. Beim Zylinder 21 ist ausserdem ein Einlass der Druckluft und das Auslassventil 24 angeordnet.

Die Druckluft wird ausgehend von einem Druckversorgungsmittel 40, einem einem Manometer 41, einem Sicherheits-Reduzierventil 42, einem Regelventil 43, einem Betriebsdruckmanometer 44 und einem Ventil 50 durch die Leitung 23 in den Einlass des Zylinders 21 geführt.

Für das Druckversorgungsmittel 40 kann eine Gasdruckflasche mit einem Gasdruck zwischen 4 und 10 bar verwendet werden, mittels welcher das Pumpenaggregat niederdruckseitig gespiesen wird. Die Gasdruckflasche ist mit Vorteil aus einer Kunststoff-Flasche gefertigt, so dass diese leicht von Hand getragen werden kann.

Mittels eines üblicherweise vorgesehenen Fusspedals 49 werden die Pumpen 40, 20 ausgeschaltet oder in Betrieb gesetzt und dementsprechend der Wasserstrahl erzeugt. Mit diesem Fusspedal 49, das mit Anschlusskupplungen 46 an die Steuerleitung 47 versehen ist, wird ein Zweiwegventil 45 betätigt, welches im dargestellten Schliesszustand bewirkt, dass das Ventil 50 geschlossen und das Druckentlastungsventil 35 den Wasserdruck abbaut und somit kein Wasserstrahl fliesst. Demgegenüber wird bei gedrücktem Fusspedal 49 und damit dem geöffneten Ventil 45 das Ventil 50 geöffnet bzw. das Ventil 35 geschlossen. Dies bewirkt eine Einschaltung des Pumpenaggregates 20 und eine Förderung des Wassers zur Düse. Beim Loslassen des Fusspedals 49 schliesst das Ventil 50 selbsttätig und schaltet das Gerät automatisch auf einen drucklosen Zustand.

Die nicht näher gezeigte, den Wasserstrahl erzeugende Düse mit dem Handgriff für die eigentliche Behandlung, sei es für das Herausschneiden von Gewebe oder für die Wundbehandlung, ist aus einem hochwertigen Metall hergestellt. Hieraus ergibt sich der weitere Vorteil im Rahmen der Erfindung, dass diese Düse mehrmals verwendet werden kann und damit geringere Unterhaltskosten entstehen, als wenn diese für einen Einmalgebrauch ausgelegt ist.

Die Erfindung ist mit den oben erläuterten Ausführungsbeispielen ausreichend dargetan. Sie liesse sich jedoch noch in anderen Varianten darstellen. So könnte das Pumpenaggregat auch anders als dargestellt ausgebildet sein. Als Druckversorgungsmittel könnte im Prinzip auch eine elektrisch betriebene Pumpe verwendet werden.

## Patentansprüche

1. Einrichtung zur Erzeugung eines Flüssigkeitsstrahles für das Entfernen von biologischem Gewebe, mit einem Gerät (11), in welchem ein Pumpenaggregat (20) zur Erzeugung eines Hochdruckes der Flüssigkeit in einem Bereich bis zu 800 bar angeordnet ist, an welches ein Druckversorgungsmittel (40) und über eine flexible Verbindungsleitung eine den Flüssigkeitsstrahl erzeugende Düse anschliessbar ist, welche bei der Austrittsöffnung einen Lochdurchmesser zwischen 20 und 120 Mikrometern aufweist, **dadurch gekennzeichnet, dass**
das Gerät und das separat zu diesem vorgesehene Druckversorgungsmittel (40) für das Pumpenaggregat jeweils ein solches Gewicht aufweisen, dass sie sich für eine mobile Anwendung eignen und demgemäss jeweils von Hand tragbar sind, wobei das Pumpenaggregat (20) als Differentialkolbenpumpe ausgebildet ist.

2. Einrichtung nach Anspruch 1, **dadurch gekennzeichnet, dass** das Druckversorgungsmittel (40) derart ausgebildet ist, dass es unabhängig von einer Netzverbindung, sei es das elektrische Stromnetz oder einem Gasdruck-Netz arbeitet.

3. Einrichtung nach Anspruch 2, **dadurch gekennzeichnet, dass** als Druckversorgungsmittel (40) eine Gasdruckflasche mit einem Gasdruck zwischen 4 und 10 bar verwendbar ist, mittels welcher das Pumpenaggregat niederdruckseitig betrieben wird.

4. Einrichtung nach Anspruch 2, **dadurch gekennzeichnet, dass** die Gasdruckflasche für einen leichten manuellen Transport einen Kunststoffflasche aufweist.

5. Einrichtung nach einem der vorhergehenden Ansprüche 1 bis 4, **dadurch gekennzeichnet, dass** das Pumpenaggregat (20) aus einem Niederdruckteil mit einem Anschluss für die Druckluftversorgung und einem Hochdruckteil mit den Anschlusssteckern (30, 31) für die Flüssigkeit und die Düse besteht.

6. Einrichtung nach einem der vorhergehenden Ansprüche 1 bis 6, **dadurch gekennzeichnet, dass** die den Wasserstrahl erzeugende Düse mit dem Handgriff für die eigentliche Behandlung, sei es für das Herausschneiden von Gewebe oder für die Wundbehandlung, aus einem hochwertigen Metall hergestellt ist, so dass diese Düse mehrmals verwendbar ist.

7. Einrichtung nach einem der vorhergehenden Ansprüche 1 bis 6, **dadurch gekennzeichnet, dass** beim hochdruckseitigen Ausgang des Pumpenaggregates (20) ferner ein Druckentlastungsventil (35) vorgesehen ist, welches in Wirkverbindung mit einem Ventil (50) zum Öffnen und Schliessen der Druckversorgungsleitung zur Pumpe steht.

## Claims

1. Device for producing a fluid jet used especially to remove biological tissue, with an appliance (11) in which a pump set (20) is arranged for producing high liquid pressure within a range of up to 800 bars to which a pressure supplying means (40) and, by means of a flexible connecting line, a nozzle producing the fluid jet can be connected, the diameter of the nozzle outlet measuring between 20 and 120 micrometers, **characterised in that**
the weight of the appliance and the separate pressure supplying means (40) envisaged for the pump set is such that each is suitable especially for portable use and can thus be carried by hand, whereby the pump set (20) is designed as a differential piston pump.

2. Device according to claim 1, **characterised in that** the pressure supplying means (40) is so designed that it operates independent of the mains whether it is a case of electrical or gas pressure mains.

3. Device according to claim 2, **characterised in that** a compressed gas bottle containing gas at a pressure between 4 and 10 bar can be used as pressure supplying means (40), to operate the pump set at the low pressure side.

4. Device according to claim, 2, **characterised in that** the compressed gas bottle is a plastic bottle to make it light and easy to carry by hand.

5. Device according to one of the preceding claims 1 to 4, **characterised in that** the pump set (20) consists of a low pressure section with a connection for the compressed air apply and a high pressure section with connecting plugs (30, 31) for the fluid and the nozzle.

6. Device according to one of the preceding claims 1 to 6, **characterised in that** the nozzle producing the water jet with the handle for the actual operation is made of a high-quality metal regardless of whether it is intended for cutting away tissue or for treating wounds, so that this nozzle can be used many times.

7. Device according to one of the preceding claims 1 to 6, **characterised in that**, furthermore, a pressure relief valve (35) is provided at the high-pressure outlet at the pump set (20), which co-acts with a valve (50) to open and close the pressure supplying line to the pump.

## Revendications

1. Dispositif de production d'un jet de liquide pour éliminer du tissu biologique, comprenant un appareil (11) dans lequel est monté un groupe (20) de pompage de production d'une haute pression du liquide dans une plage allant jusqu'à 800 bars, auquel peut être raccordé un moyen (40) d'alimentation sous pression et, par un conduit souple de liaison, une buse produisant le jet de liquide qui a, à l'ouverture de sortie, un diamètre de trou compris entre 20 et 120 microns, **caractérisé en ce que**
l'appareil et le moyen (40) d'alimentation en pression, prévu séparément de celui-ci, du groupe de pompage ont respectivement un poids tel qu'ils conviennent pour une application mobile et peuvent donc être portés respectivement à la main, le groupe (20) de pompage étant constitué sous la forme d'une pompe à piston différentiel.

2. Dispositif suivant la revendication 1, **caractérisé en ce que** le moyen (40) d'alimentation sous pression est tel qu'il fonctionne indépendamment d'une liaison au réseau, que ce soit le réseau de courant électrique ou un réseau de gaz comprimé.

3. Dispositif suivant la revendication 2, **caractérisé en ce que** l'on peut utiliser comme moyen (40) d'alimentation sous pression une bouteille de gaz comprimé ayant une pression du gaz comprise entre 4 et 10 bars, au moyen de laquelle le groupe de pompage est mis en fonctionnement du côté de la pression.

4. Dispositif suivant la revendication 2, **caractérisé en ce que** la bouteille de gaz comprimé a, pour en faciliter le transport à la main, une anse en matière plastique.

5. Dispositif suivant l'une des revendications précédentes 1 à 4, **caractérisé en ce que** le groupe (20) de pompage est constitué d'une partie basse pression ayant un raccord pour l'alimentation en air comprimé et d'une partie haute pression ayant des connecteurs (30, 31) de raccordement pour le liquide et la buse.

6. Dispositif suivant l'une des revendications précédentes 1 à 6, **caractérisé en ce que** la buse produisant le jet d'eau ayant la poignée pour le traitement proprement dit, que ce soit pour enlever du tissu pour le traitement d'une blessure, est en un métal précieux, de sorte que l'on peut réutiliser cette buse plusieurs fois.

7. Dispositif suivant l'une des revendications précédentes 1 à 6, **caractérisé en ce que** pour une sortie du côté haute pression du groupe (20) de pompage, il est prévu en outre une soupape (35) de détente de la pression qui coopère avec une vanne (50) d'ouverture et de fermeture du conduit d'alimentation sous pression de la pompe.
